# EUROPÄISCHE PATENTSCHRIFT

(11) **EP 2 268 405 B1**
(45) Veröffentlichungstag und Bekanntmachung des Hinweises auf die Patenterteilung: **04.01.2017**
(21) Anmeldenummer: 09714713.6
(22) Anmeldetag: 26.02.2009
(51) Int. Cl.: G01N 33/49, B01L 3/00

(54) **VORRICHTUNG ZUR PLASMASEPARATION**
APPARATUS FOR THE SEPARATION OF PLASMA
DISPOSITIF DE SÉPARATION DE PLASMA

(30) Priorität: 27.02.2008 DE 102008011339
(43) Veröffentlichungstag der Anmeldung: 05.01.2011
(73) Patentinhaber: Boehringer Ingelheim Microparts Gmbh, 44227 Dortmund (DE)
(72) Erfinder: BLANKENSTEIN, Gert, 44141 Dortmund (DE); BARTOS, Holger, 44287 Dortmund (DE); PETERS, Ralf-Peter, 51467 Bergisch-Gladbach (DE); SCHOEN, Christian, 44229 Dortmund (DE)
(74) Vertreter: Simon, Elke Anna Maria
(86) Internationale Anmeldenummer: PCT/EP2009/001383
(87) Internationale Veröffentlichungsnummer: WO 2009/106331

(56) Entgegenhaltungen:
- EP-A- 1 201 304
- EP-A- 1 495 799
- EP-A- 1 531 003
- EP-A- 1 559 676
- EP-A- 1 685 900
- EP-A- 1 714 698
- WO-A-03/103835
- WO-A-2005/119211
- DE-A1- 19 753 849
- US-A- 5 922 604
- US-A1- 2004 077 103

## Beschreibung

Die vorliegende Erfindung betrifft eine Vorrichtung zur Aufnahme von Blut und Abtrennung von Blutbestandteilen, wie Blutplasma, als Probenflüssigkeit.

Die vorliegende Erfindung befasst sich mit mikrofluidischen Systemen bzw. Vorrichtungen. Die nachfolgenden Ausführungen beziehen sich auf Vorrichtungen, bei denen Kapillarkräfte wirken und insbesondere für die Funktion entscheidend sind.

Aus der US 4,906,439 A und der WO 01/24931 A1 sind jeweils Vorrichtungen zur Abtrennung von Blutplasma aus Blut bekannt, wobei eine Vielzahl von nutartigen bzw. kapillarartigen Einzelkanälen zur Aufnahme und Ableitung des Blutplasmas vorgesehen sind. Hier tritt der Nachteil auf, dass sich die Kanäle unterschiedlich schnell oder gar nicht mit der Probenflüssigkeit in Form von Blutplasma füllen. Dementsprechend kann keine einheitliche Flüssigkeitsfront erreicht werden. Dies ist hinsichtlich der Diagnostik nachteilig, da keine definierte Menge gleichzeitig zur Verfügung steht oder beispielsweise nicht gleichzeitig Trockenchemikalien oder dergleichen in dem gewünschten bzw. erforderlichen Maß von der Probenflüssigkeit gelöst werden können.

Aus der WO 2005/119211 A1 ist eine Vorrichtung zur Abtrennung von Blutplasma aus Blut mittels einer Trennabrichtung, insbesondere einer Membran bekannt, wobei das abgetrennte Blutplasma per Kapillarkraft in einem Kanal abgeleitet wird.

Der vorliegenden Erfindung liegt die Aufgabe zugrunde, eine verbesserte Vorrichtung und ein verbessertes Verfahren zur Aufnahme von Blut und Abtrennung von Blutbestandteilen, wie Blutplasma, als Probenflüssigkeit bereitzustellen, wobei ein optimiertes Füllen des Kanals mit Probenflüssigkeit ermöglicht wird und wobei ein Kapillarkontakt zwischen dem Seperationselement und dem Kanal hergestellt wird und vorzugsweise die diagnostischen bzw. Untersuchungsmöglichkeiten verbessert werden.

Eine grundlegende Idee der vorliegenden Erfindung liegt darin, eine Einrichtung zum Herstellen eines fluidischen Kapillarkontaktes zwischen Seperationselementen, insbesondere Membranen und einem Leitungskanal vorzusehen. Dies gestattet ein optimales, schnelles und gleichmäßiges Füllen des Kanals und verhindert unerwünschte Lufteinschlüsse.
Vorzugsweise wird die Probenflüssigkeit durch Kapillarkräfte in einem Kanal aufgenommen, der zumindest an einer Schmalseite oder Längsseite offen ausgebildet ist, so dass ein seitlicher Flüssigkeitsstopp für die Probenflüssigkeit im Kanal gebildet wird und die Probenflüssigkeit seitenwandfrei im Kanal führbar ist. Insbesondere schließt sich hierbei eine Ausnehmung seitlich an die offene Seite des Kanals an.

So kann auf einfache Weise ein Vorschiessen der Probenflüssigkeit - also schnelleres Füllen des Kanals - im Bereich einer ansonsten vorhandenen Seitenwand verhindert werden. Dies ermöglicht eine Vergleichmäßigung der Füllgeschwindigkeit über den gesamten Kanalquerschnitt, so dass eine zumindest im Wesentlichen gleichmäßige bzw. gradlinige Flüssigkeitsfront beim Füllen des Kanals erreichbar ist.
Die seitlich offene Ausbildung des Kanals gestattet eine verbesserte, insbesondere optimale Entlüftung beim Füllen des Kanals mit Probenflüssigkeit.
Außerdem gestattet die seitenwandfrei gehaltene bzw. geführte Oberfläche der Probenflüssigkeit eine unmittelbare Untersuchung der Probenflüssigkeit, insbesondere durch Einkopplung von Licht, ohne eine ansonsten vorhandene Seitenwand o. dgl.
Vorzugsweise ist die Ausnehmung grabenartig ausgebildet und umgibt den insbesondere allseitig seitlich offenen Kanal vollständig. So können insbesondere bei sehr feinen Strukturen die ansonsten beim Übergang von Flachseiten zu Schmalseiten auftretenden Innenkanten mit besonders hohen Kapillarkräften insgesamt vermieden werden. Jedoch gilt entsprechendes auch bei einer nur abschnittsweise seitlich offenen Ausbildung des Kanals. Alternativ kann die sich seitlich an den Kanal anschließende Ausnehmung oder Seitenwandung auch mit Probenflüssigkeit oder einer sonstigen Flüssigkeit füllbar sein. Hierbei ist die Ausnehmung oder Seitenwandung dann derart - insbesondere hinsichtlich ihrer Größe, Krümmungen oder ihrem Benetzungsverhalten - oder durch Leitelemente gestaltet, dass die Füllgeschwindigkeit des Kanals mit der Probenflüssigkeit größer oder gleich der Füllgeschwindigkeit der Ausnehmung oder entlang der Seitenwandung jeweils in Füllrichtung - insbesondere Längsrichtung - des Kanals ist. So kann ebenfalls ein seitliches Vorschiessen der Flüssigkeitsfront beim Füllen mit Probenflüssigkeit vermieden werden.

Ein weiteres vorschlagsgemäßes Verfahren zur Bestimmung eines Parameters im Blutplasma bzw. eines Blutbestandteils zeichnet sich dadurch aus, dass in einem mikrofluidischen System unmittelbar nach dem Zurückhalten bzw. Abtrennen von Blutzellen direkt eine Bestimmung eines Bestandteils bzw. Parameters des Blutplasmas mittels einer Chemikalie oder mehreren Chemikalien erfolgt. Dies gestattet bei einfachem und kompaktem Aufbau eine schnelle und preisgünstige Analyse bzw. Bestimmung des Parameters.

Weitere Vorteile, Merkmale, Eigenschaften und Aspekte der vorliegenden Erfindung ergeben sich aus den Ansprüchen und der folgenden Beschreibung bevorzugter Ausführungsformen anhand der Zeichnung. Es zeigt:
Fig. 1 einen schematischen Schnitt einer vorschlagsgemäßen Vorrichtung gemäß einer ersten Ausführungsform;
Fig. 2 eine schematische Draufsicht eines Trägers der gefüllten Vorrichtung gemäß Fig. 1;
Fig. 3 einen schematischen Schnitt der Vorrichtung entlang Linie III-III gemäß Fig. 2;
Fig. 4 einen schematischen Längsschnitt der Vorrichtung entlang Linie IV-IV gemäß Fig. 2;
Fig. 5 eine schematische Draufsicht eines Trägers einer vorschlagsgemäßen Vorrichtung gemäß einer zweiten Ausführungsform;
Fig. 6 eine schematische Draufsicht eines Trägers einer vorschlagsgemäßen Vorrichtung gemäß einer dritten Ausführungsform;
Fig. 7 eine Ausschnittsweise schematische Draufsicht eines Trägers einer vorschlagsgemäßen Vorrichtung gemäß einer vierten Ausführungsform;
Fig. 8 einen Ausschnittsweisen, schematischen Schnitt der Vorrichtung entlang Linie VIII-VIII gemäß Fig. 7;
Fig. 9 einen schematischen Längsschnitt einer vorschlagsgemäßen Vorrichtung gemäß einer fünften Ausführungsform; und
Fig. 10 einen schematischen Schnitt einer vorschlagsgemäßen Vorrichtung gemäß einer sechsten Ausführungsform;
Fig. 11A, B, C und Fig. 12 Ausführungsform mit gewölbten Membrananordnung;
Fig. 13, Fig. 14, Fig. 15 und Fig. 16 Ausführungen mit einer durch einen Stempel gewölbten Membran;
Fig. 17 und Fig. 18 Ausführungsformen mit gewölbten Trägerboden;
Fig. 19 und Fig. 19A, B, C Ausführungen mit einem Einlegeeinsatz;
Fig. 20 eine Ausführung mit einer Entlüftung;
Fig. 21 eine Ausführung mit verschweißter Membran und
Fig. 22, Fig. 22A, Fig. 23 und Fig. 23A eine Aufweitung des Kanaleinlassquerschnittes.

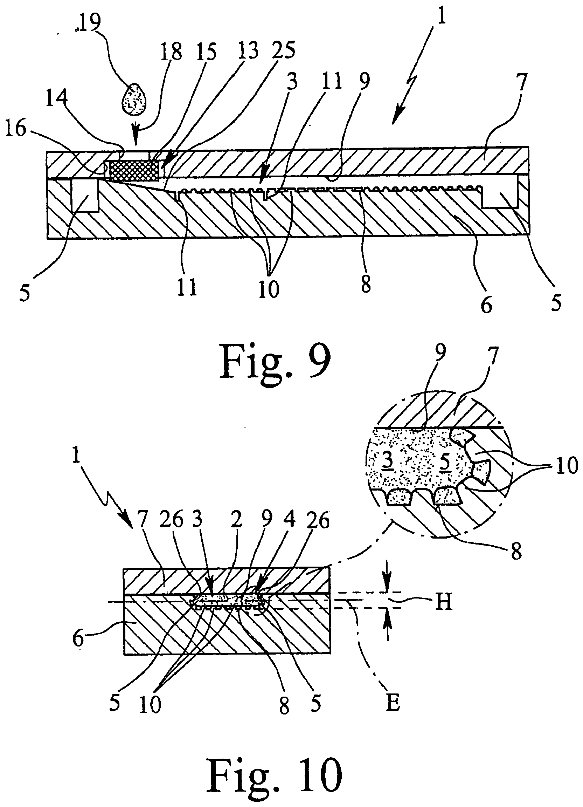

In den Figuren werden für gleiche oder ähnliche Teile dieselben Bezugszeichen verwendet, wobei entsprechende oder vergleichbare Eigenschaften und Vorteile erreicht werden, auch wenn eine wiederholte Beschreibung weggelassen ist.

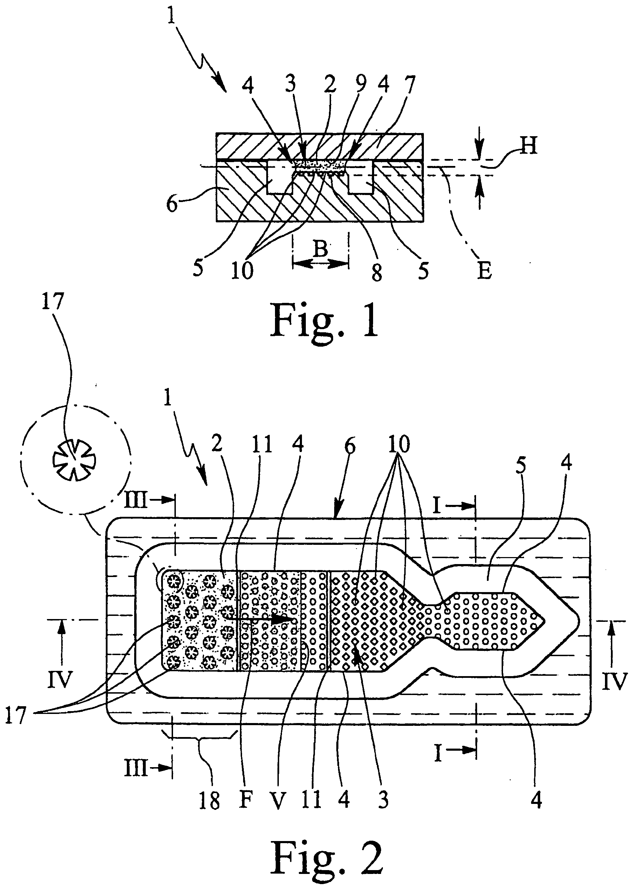

Fig. 1 zeigt in einem schematischen Schnitt eine erste Ausführungsform einer vorschlagsgemäßen Vorrichtung (1) zur Aufnahme und/oder Diagnostik einer Probenflüssigkeit (2), insbesondere Blutplasma o. dgl. Die Vorrichtung (1) weist einen die Probenflüssigkeit (2) durch Kapillarkräfte aufnehmenden Kanal (3) auf. Der Kanal (3) ist zumindest an einer Schmalseite bzw. Längsseite (4), beim Darstellungsbeispiel auf beiden Schmal- bzw. Längsseiten (4), offen ausgebildet, wie in Fig. 1 angedeutet.
Seitlich schließt sich an die offenen Seiten (4) eine Ausnehmung (5) an, die beim Darstellungsbeispiel vorzugsweise nut- bzw. grabenartig ausgebildet ist.
So wird ein seitlicher Flüssigkeitsstopp für die Probenflüssigkeit (2) - also ein durch Kapillarkräfte nicht überwindbares Strömungshindernis - im Kanal (3) gebildet und ist die Probenflüssigkeit (2) seitenwandfrei entlang der offenen Seiten (4) im Kanal (3) führbar. Beim Darstellungsbeispiel weist die Vorrichtung (1) einen Träger (6) und eine zugeordnete Abdeckung (7) auf, zwischen denen der Kanal (3) und die Ausnehmung (5) gebildet sind. Bedarfsweise ist lediglich der Träger (6) zur Bildung der erforderlichen Strukturen ausgenommen und die Abdeckung (7) eben, vorzugsweise zumindest im Wesentlichen ausnehmungsfrei, ausgebildet. Jedoch kann dies auch umgekehrt sein. Bedarfsweise können aber auch sowohl der Träger (6) als auch die Abdeckung (7) ausgenommen und/oder mit Vorsprüngen zur Bildung der gewünschten Strukturen und ggf. zur Aufnahme von nicht dargestellten Chemikalien, Reagenzien, Untersuchungseinrichtungen o. dgl. ausgebildet sein.
Die Ausnehmung (5) schließt sich vorzugsweise scharfkantig an den Kanal (3) an, wie in Fig. 1 angedeutet. Beim Darstellungsbeispiel ist die Ausnehmung (5) nur im Träger (6) gebildet, erstreckt sich bei der Darstellung gemäß Fig. 1 also im Wesentlichen nur nach unten bezüglich einer seitlichen Projektion des Kanals (3). Die Ausnehmung (5) kann sich jedoch wahlweise auch nach oben oder auf beide Seiten der seitlichen Projektion des Kanals (3) - also insbesondere nach oben und nach unten - erstrecken.
Die im Querschnitt vorzugsweise rechteckige Ausnehmung (5) führt zu einer derartigen, insbesondere stufigen bzw. plötzlichen Querschnittsvergrößerung, dass sich die Kapillarkräfte derartig verringern, dass der genannte Flüssigkeitsstopp für die Probenflüssigkeit (2) im Übergang vom Kanal (3) zur Ausnehmung (5) hin gebildet wird, wie in Fig. 1 angedeutet.
Der Kanal (3) wird vorzugsweise von nur zwei gegenüberliegenden, insbesondere im wesentlichen ebenen Flächen bzw. Flachseiten (8) und (9), die beim Darstellungsbeispiel durch den Träger (6) bzw. die Abdeckung (7) gebildet sind und parallel verlaufen, begrenzt bzw. gebildet. Bedarfsweise kann daher die Ausnehmung (5) auch ganz entfallen und der Kanal (3) beispielsweise durch zwei geeignete Stege o. dgl. mit geeignetem Abstand zur Erzeugung der gewünschten Kapillarkräfte gebildet sein.
Fig. 2 zeigt in einer schematischen Draufsicht den Träger (6) der Vorrichtung (1) ohne Abdeckung (7), jedoch mit teilweiser Füllung durch die Probenflüssigkeit (2) bis zur Flüssigkeitsfront V. Die Ausnehmung (5) erstreckt sich beim Darstellungsbeispiel entlang der offenen Seite(n) (4) des Kanals (3), vorzugsweise zumindest entlang gegenüberliegender, offener Längsseiten (4). Weiter ist beim Darstellungsbeispiel der Kanal (3) allseitig seitlich offen ausgebildet und die Ausnehmung (5) dementsprechend umlaufend ausgebildet.
Der Kanal (3) ist also allseitig von der Ausnehmung (5) umgeben.
Vorzugsweise schließt sich die Ausnehmung (5) an diejenigen Schmalseiten bzw. Längsseiten (4) des Kanals (3) an, die sich zumindest im Wesentlichen parallel zur Hauptfüllrichtung F des Kanals (3) mit Probenflüssigkeit (2), wie in Fig. 2 angedeutet, erstrecken. Folglich verläuft die Ausnehmung (5) vorzugsweise zumindest abschnittsweise parallel zur Hauptfüllrichtung F.
Gemäß einer anderen, später anhand von Fig. 10 erläuterten Variante ist es auch möglich, dass sich die Ausnehmung (5) mit der Probenflüssigkeit (2) oder einer anderen, sich mit der Probenflüssigkeit (2) insbesondere nicht mischenden Flüssigkeit, wie Öl oder dergleichen, füllt. In diesem Fall ist die Ausnehmung (5) jedoch derart gestaltet, dass deren Füllgeschwindigkeit maximal so groß wie die Füllgeschwindigkeit des Kanals (3) ist, um ein möglichst gleichmäßiges Füllen mit Probenflüssigkeit (2) zu erreichen. Die Füllgeschwindigkeiten beziehen sich dabei jeweils auf das Füllen bzw. Voranschreiten der Flüssigkeitsfront V in die Hauptfüllrichtung F.
Alternativ kann die Ausnehmung (5) vor Einleiten der Probenflüssigkeit (2) auch nur mit der anderen Flüssigkeit gespült werden.
Der Kanal (3) weist vorzugsweise einen im Wesentlichen rechteckigen und/oder flachen Querschnitt, insbesondere quer zur Hauptfüllrichtung F, auf.
Die in Fig. 1 angedeutete Höhe H des Kanals (3) - also der Abstand der den Kanal (3) begrenzenden, vorzugsweise parallelen Flächen (8) und (9) - beträgt maximal 2000 Mikrometer, vorzugsweise höchstens 500 Mikrometer, insbesondere etwa 50 bis 200 Mikrometer. Die Ausnehmung (5) führt vorzugsweise zu einer stufigen bzw. plötzlichen Vergrößerung der Höhe H und dadurch zur Bildung des gewünschten Flüssigkeitstopps. Insbesondere ist die Höhe H der Ausnehmung (5) mindestens doppelt so groß wie die Höhe H des Kanals (3).
Die Breite B des Kanals (3) beträgt vorzugsweise etwa 100 bis 5000 Mikrometer, insbesondere etwa 200 bis 4000 Mikrometer.
Die Höhe H des Kanals (3) ist wesentlich geringer, insbesondere mindestens um den Faktor 5 oder 10, als die Breite B des Kanals (3).
Das Aufnahmevolumen des Kanals (3) beträgt vorzugsweise weniger als 1 ml, insbesondere weniger als 100 [mu]l, besonders bevorzugt maximal 10 [mu]l.
Die Vorrichtung (1) bildet also ein mikrofluidisches System. Insbesondere dient die Vorrichtung (1) der mikrofluidischen Diagnostik für medizinische oder nicht-medizinische Zwecke bzw. sonstige Untersuchungen.
Der Kanal (3) und damit dessen Hauptfüllrichtung F und Haupterstreckungsebene E verlaufen in Gebrauchslage vorzugsweise zumindest im Wesentlichen horizontal. Je nach Verwendungszweck oder konstruktiver Lösung ist jedoch auch eine andere Ausrichtung möglich, zumal die Aufnahme bzw. das Füllen des Kanals (3) mit Probenflüssigkeit (2) vorzugsweise zumindest primär nur durch Kapillarkräfte bestimmt bzw. bewirkt wird.
So kann die Hauptfüllrichtung F beispielsweise horizontal oder geneigt verlaufen, während die Haupterstreckungsebene E beispielsweise vertikal verläuft, so dass der Kanal (3) also hochkant ausgerichtet ist.
Der Kanal (3) bildet vorzugsweise mindestens einen Speicher für die Probenflüssigkeit (2), insbesondere zur Diagnostik. Ggf. kann der Kanal (3) eine nicht dargestellte Chemikalie, insbesondere eine Trockenchemikalie o. dgl., enthalten. Jedoch können Untersuchungen der Probenflüssigkeit (2) auch auf sonstige Weise vorgenommen werden.
Beim Darstellungsbeispiel weist der Kanal (3) mindestens ein Leitelement zur Beeinflussung, insbesondere Vergleichmäßigung, des Füllens mit der Probenflüssigkeit (2) auf.
Gemäß einer Ausbildungsvariante weist der Kanal (3) vorzugsweise regelmäßig verteilte Erhöhungen (10) als Leitelemente auf. Diese sind insbesondere in Reihen quer, vorzugsweise senkrecht, oder längs zur Hauptfüllrichtung F, insbesondere abwechselnd quer versetzt, angeordnet. Die Erhöhungen (10) sind die Reihen in Hauptfüllrichtung F versetzt. So kann erreicht werden, dass die Probenflüssigkeit (2) den Kanal (3) reihenweise - also Reihe für Reihe - füllt und dadurch mit einer im wesentlichen gradlinigen Flüssigkeitsfront V in Hauptfüllrichtung F fortschreitet.
Bedarfsweise kann die Flächendichte, der Abstand und/oder die Größe der Erhöhungen (10) variieren, insbesondere in Abhängigkeit von der jeweiligen Entfernung zu einem in Fig. 1 und
Fig. 2 nicht dargestellten Einlass für die Probenflüssigkeit (2) in den Kanal (3).
Die Erhöhungen (10) sind vorzugsweise steg-, höcker- oder säulenartig, insbesondere mit runder oder polygonaler Grundfläche, ausgebildet. Stattdessen können aber auch Vertiefungen vorgesehen sein.
Alternativ oder zusätzlich kann der Kanal (3) mindestens einen Graben (11) oder einen Steg als Leitelement aufweisen, der quer oder längs zur Hauptfüllrichtung F des Kanals (3) verläuft. Der vorzugsweise vorgesehene nutartige, im Querschnitt insbesondere rechteckige oder halbrunde Graben (11) weist eine wesentlich geringere Tiefe als die Ausnehmung (5) auf und bildet daher einen nur temporären Flüssigkeitsstopp zur Vergleichmäßigung der Flüssigkeitsfront V. So kann erreicht werden, dass die Probenflüssigkeit (2) erst nach Füllen des Kanals (3) über seinen gesamten Querschnitt den Graben (11) und anschließend den nachfolgenden Kanalbereich füllt.
Hervorzuheben ist, dass durch die Kombination der seitenwandlosen Führung der Probenflüssigkeit (2) und der Leitelemente ein hochgradig gleichmäßiges Füllen des Kanals (3) durch Kapillarkräfte mit zumindest im wesentlichen gradliniger bzw. senkrecht zur Hauptfüllrichtung F verlaufender Flüssigkeitsfront V erreichbar ist.
Alternativ kann der Kanal (3) und/oder ein davon gebildeter Speicher, Sammelraum,
Sammelbereich o. dgl. auch zumindest im Wesentlichen glatt bzw. eben, also insbesondere ohne Leitelemente, ausgebildet sein.

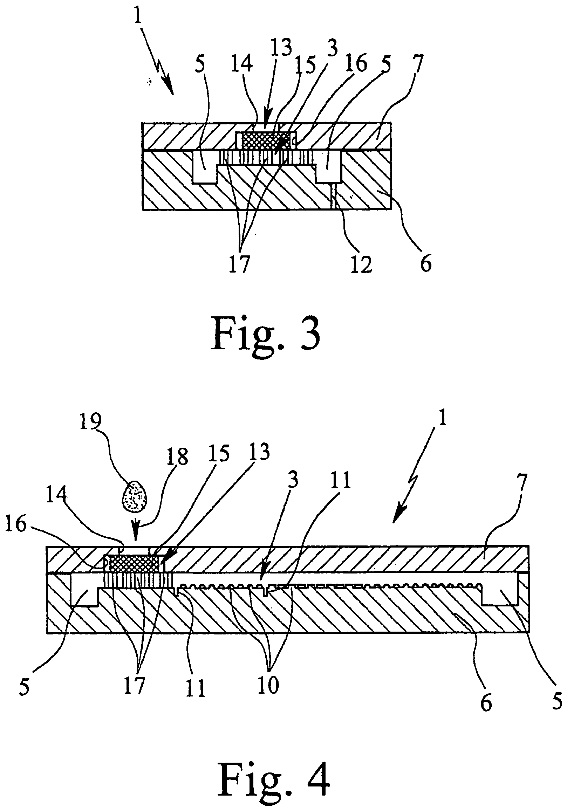

Fig. 3 zeigt einen weiteren schematischen Schnitt der Vorrichtung (1) mit der Abdeckung (7) entlang Linie III-III gemäß Fig. 2.
Die Vorrichtung (1) weist mindestens eine dem Kanal (3) zugeordnete Entlüftung (12) auf, die nicht unmittelbar an den Kanal (3), sondern an die Ausnehmung (5) angeschlossen ist. So ist kein zusätzlicher Flüssigkeitsstopp für die Entlüftung (12), um ein Austreten von Probenflüssigkeit (2) durch die Entlüftung (12) zu verhindern, erforderlich. Die vorzugsweise allseitig seitlich offene Ausbildung des Kanals (3) gestattet ein optimales Entlüften beim Füllen des Kanals (3) mit der Probenflüssigkeit (2), so dass unerwünschte Lufteinschlüsse sicher vermieden werden können.
Die Probenflüssigkeit (2) ist dem Kanal (3) vorzugsweise senkrecht zur Kanalerstreckung E, insbesondere in Gebrauchslage vertikal, zuführbar.
Die Vorrichtung (1) weist eine Zuführeinrichtung (13) zur Aufnahme und Zuführung von Probenflüssigkeit (2) zum Kanal (3) auf. Beim Darstellungsbeispiel weist die Zuführeinrichtung (13) eine Öffnung, insbesondere Durchbrechung (14), in der Abdeckung (7), vorzugsweise zur Aufnahme von Blut o. dgl., sowie eine Trenneinrichtung (15), wie einen Filter, eine Membran o. dgl., zur Abtrennung von Blutplasma als Probenflüssigkeit (2) auf. Die Trenneinrichtung (15) ist beim Darstellungsbeispiel in eine zum Träger (6) hin offene Aussparung (16) in der Abdeckung (7) eingesetzt und deckt die Durchbrechung (14) ab. Vorzugsweise ist die Trenneinrichtung (15) mit der Abdeckung (7) fest verbunden, beispielsweise verschweißt, verklebt oder von dieser kraft- oder formflüssig gehalten.
Die Trenneinrichtung (15) steht - beim Darstellungsbeispiel mit einer Flachseite - unmittelbar mit dem Kanal (3) in Kontakt, insbesondere liegt die Trenneinrichtung (15) auf vorzugsweise säulenartigen Strukturen (17) o. dgl. im Kanal (3) in einem Zuführbereich (18) des Kanals (3) auf. Die Strukturen (17) sind vorzugsweise mit keilartigen Ausnehmungen o. dgl. versehen, um das Blutplasma bzw. die Probenflüssigkeit (2) durch Kapillarkräfte zu der der Trenneinrichtung (15) gegenüberliegenden Kanalfläche - hier zu der vom Träger (6) gebildeten Bodenfläche (8) des Kanals (3) - zu leiten und so eine vollständige Füllung zwischen Bodenfläche (8) und Abdeckung (7) bzw. des Zuführbereichs (18) mit Probenflüssigkeit (2) zu bewirken.
Die Strukturen (17) bilden eine Fülleinrichtung zum (vollständigen) Füllen des Kanals (3) zwischen Abdeckung (7) und Bodenfläche (8) mit Probenflüssigkeit (2).
Diese Fülleinrichtung kann jedoch auch in sonstiger Weise ausgebildet sein, wie später noch anhand der fünften Ausführungsform erläutert.
Anschließend wird die Probenflüssigkeit (2) - beim Darstellungsbeispiel nach Überwindung des ersten Grabens (11) - durch Kapillarkräfte weiter in den Kanal (3) gesaugt, wie durch die Hauptfüllrichtung F in Fig. 2 angedeutet.

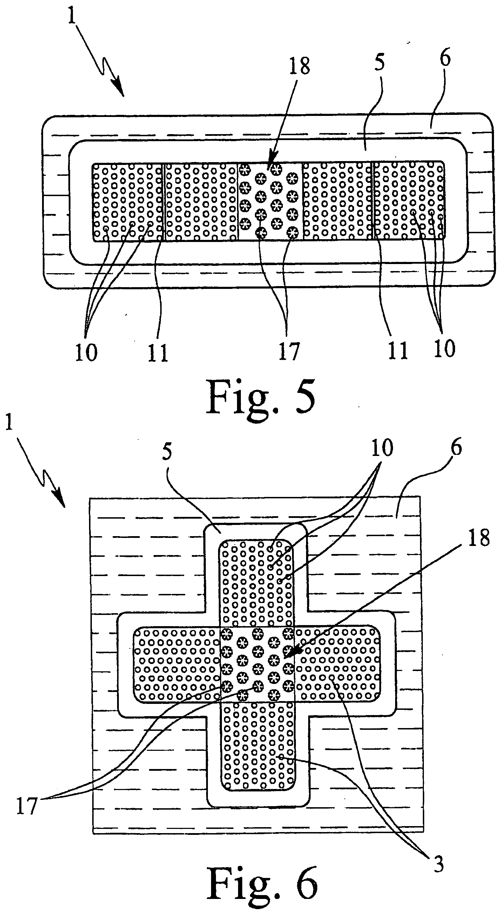

Fig. 4 zeigt in einem schematischen Längsschnitt den bevorzugten Aufbau der vorschlagsgemäßen Vorrichtung (1) gemäß der ersten Ausführungsform, wobei zur Veranschaulichung ein zugeführter Tropfen Blut (19) angedeutet sind.
Die Trenneinrichtung (15) kann bedarfsweise eine Chemikalie, insbesondere eine Trockenchemikalie, enthalten, insbesondere um die beim Darstellungsbeispiel gewünschte Abtrennung von Blutplasma als Probenflüssigkeit (2) vom Blut (19) zu ermöglichen oder zu unterstützen und/oder bedarfsweise eine Lysierung von Zellen zu ermöglichen. Die Abtrennung bzw. Weiterleitung erfolgt insbesondere ausschließlich durch Kapillarkräfte. Vorzugsweise schließt sich an die Zuführeinrichtung (13) nur ein einziger Kanal (3) zur Aufnahme oder Ableitung der Probenflüssigkeit (2) an. Hierbei ist der Kanal (3) im Sinne einer Einzelkapillare zu verstehen. Bedarfsweise kann der Kanal (3) jedoch in unterschiedliche Richtungen oder zu unterschiedlichen Bereichen führen oder sich verzweigen, wie nachfolgend unter Bezugnahme auf die zweite Ausführungsform gemäß Fig. 5 und die dritte Ausführungsform gemäß Fig. 6 erläutert.
Fig. 5 und Fig. 6 zeigen jeweils eine Draufsicht des Trägers (6) der Vorrichtung (1) gemäß der zweiten bzw. dritten Ausführungsform, jeweils ohne Abdeckung (7).
Bei der zweiten Ausführungsform gemäß Fig. 5 erstreckt sich der Kanal (3) ausgehend von der Zuführeinrichtung (13) bzw. dem Zuführbereich (18) auf entgegengesetzte Seiten bzw. in entgegengesetzte Richtungen, beispielsweise um simultan unterschiedliche Untersuchungen, Tests o. dgl. durchzuführen. Hier ergibt sich eine im Wesentlichen längliche Anordnung.
Bei der dritten Ausführungsform gemäß Fig. 6 ist eine kreuzartige Konfiguration vorgesehen. Hier erstreckt sich der Kanal (3) in vier unterschiedliche Richtungen. So können beispielsweise gleichzeitig vier ggf. unterschiedliche Untersuchungen, Test, Reaktionen o. dgl. durchgeführt werden.
Sowohl bei der zweiten Ausführungsform als auch bei der dritten Ausführungsform ist vorzugsweise wiederum die Ausnehmung (5) zur zumindest abschnittsweisen, seitenwandfreien Führung der Probenflüssigkeit (2) im Kanal (3) vorgesehen. Insbesondere umgibt die Ausnehmung (5) die gesamte Kanalkonfiguration vollständig, wobei der Kanal (3) vorzugsweise wiederum allseitig seitlich offen ausgebildet sein kann.

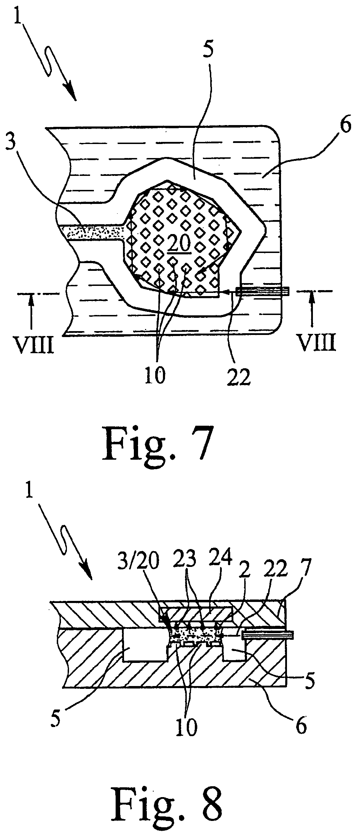

Fig. 7 und Fig. 8 zeigen eine vierte Ausführungsform der vorschlagsgemäßen Vorrichtung (1), und zwar Fig. 7 eine Draufsicht des Trägers (6) ohne Abdeckung (7) und Fig. 8 eine Schnittansicht entlang Linie VIII-VIII von Fig. 7 bei vorhandener Abdeckung (7). Der Kanal
(3) bildet hier eine Sammelkammer (20) für die Probenflüssigkeit (2). Die Sammelkammer (20) ist wiederum im Wesentlichen flach ausgebildet und weist bedarfsweise die angedeuteten Erhöhungen (10) und/oder sonstige Leitelemente o. dgl. auf.

Die Vorrichtung (1) gemäß der vierten Ausführungsform weist eine Einrichtung (21), insbesondere einen Lichtleiter o. dgl., zur Einkopplung von Licht in die Probenflüssigkeit (2), insbesondere für Fluoreszenzmessungen, auf. Das Licht trifft im Bereich einer offenen Seite (4) des Kanals (3) auf die freie Oberfläche der Probenflüssigkeit (2) und tritt aufgrund einer entsprechend steilen, vorzugsweise im wesentlichen zur Flüssigkeitsoberfläche senkrechten Auftreffrichtung in die Probenflüssigkeit (2) ein, wie durch Pfeil (22) angedeutet. Für den Lichteintritt wird also die Grenzfläche Gas (Luft)/Probenflüssigkeit (2) genutzt. So kann vermieden werden, dass das Licht durch eine ansonsten vorhandene Seitenwand geleitet werden muss und dadurch in unerwünschter Weise gestreut wird oder Fluoreszenz hervorrufen kann.
Wie in Fig. 7 angedeutet, wird der eintretende Lichtstrahl (22) vorzugsweise mehrfach durch Totalreflexion an der Grenzfläche Probenflüssigkeit (2)/Gas (Luft) reflektiert. Dies wird dadurch erreicht, dass der Winkel zwischen Flächenlot und einfallendem Lichtstrahl jeweils größer als der Grenzwinkel der Totalreflexion ist. Die Grund- bzw. Bodenfläche der Sammelkammer (20), die durch die umlaufende Ausnehmung (5) begrenzt und definiert ist, ist entsprechend gewählt, um die gewünschte Strahlführung und Totalreflexion zu erreichen, beim Darstellungsbeispiel durch eine geeignete polygonale Konfiguration.
Das eingestrahlte Licht (22) dient der Fluoreszenzbestimmung bzw. Fluoreszenzspektroskopie. Die Probenflüssigkeit (2), insbesondere darin enthaltene Marker-Moleküle o.
dgl., die beispielsweise als Chemikalie in Kanal (3) vorhanden sind und durch Probenflüssigkeit gelöst werden, werden durch eine bestimmte Wellenlänge angeregt. Dies führt zu Elektronenübergängen in den Molekülen, die nach einer gewissen Zeit unter Emission eines Photons in ihren Ursprungszustand zurückfallen. Die emittierte Strahlung ist in Fig. 8 durch Pfeile (23) angedeutet und mittels eines Detektors (24) detektierbar.
Um den Einfluss der Erhöhungen (10) oder sonstiger Leitelemente auf den einfallenden Lichtstrahl (22) auszuschließen, ist die Lichtstrahlebene oberhalb derartiger bzw. beabstandet zu derartigen Strukturen angeordnet. Des weiteren verläuft die Lichtstrahlebene zumindest im wesentlichen parallel zur Haupterstreckungsebene bzw. in der Haupterstreckungsebene E des Kanals (3) bzw. der Sammelkammer (20).
Die vorgesehene Lichteinstrahlung und Lichtführung gestatten eine weitgehend vollständige Anregung der Probenflüssigkeit (2) bzw. von darin enthaltenen Marker-Molekülen o. dgl. sowie gleichzeitig den Einsatz von Mikrostrukturen, wie den Erhebungen (10) oder sonstigen Leitelementen.
Die Erfassung der emittierten Lichtstrahlen (23) quer, insbesondere senkrecht, zur Einstrahlrichtung (22) ist hinsichtlich einer Entkopplung vom einfallenden Licht optimal.
Fig. 9 zeigt einen schematischen Längsschnitt einer fünften Ausführungsform der vorschlagsgemäßen Vorrichtung (1).
Gegenüber der ersten Ausführungsform weist hier die Fülleinrichtung zur Füllung des Kanals (3) zwischen den beiden Flachseiten (8) und (9), insbesondere zur Ableitung des Blutplasmas bzw. der Probenflüssigkeit (2) von der Trenneinrichtung (15) bzw. von der Deckfläche (9) zu der gegenüberliegenden Bodenfläche (8), um einen räumlichen Meniskus zwischen den beiden Flächen bzw. Flachseiten (8) und (9) zu bilden, alternativ oder zusätzlich zu den Strukturen (17) eine Schräge bzw. Rampe (25) auf, die die Kanalhöhe H entsprechend verringert oder gegebenenfalls sogar zu Null werden lässt. Insbesondere kann die Trenneinrichtung (15) unmittelbar mit der Rampe (25) in Kontakt stehen bzw. darauf aufliegen. Die genannte Fülleinrichtung kann auch als Einrichtung zur Deckel- und Bodenbenetzung bezeichnet bzw. verstanden werden.
Die schematische Schnittdarstellung gemäß Fig. 10 zeigt eine sechste Ausführungsform der vorschlagsgemäßen Vorrichtung (1). Hier ist die sich seitlich an den Kanal (3) anschließende Ausnehmung (5) durch die Probenflüssigkeit (2) füllbar und derart - insbesondere aufgrund einer entsprechenden Rundung ihrer Seitenwandung (26) und/oder durch Ausbildung von entsprechenden Leitelementen, wie Erhöhungen (10) oder dergleichen - ausgebildet, dass die Füllgeschwindigkeit der Ausnehmung (5) in Hauptfüllrichtung F - bei der Darstellung gemäß Fig. 10 also senkrecht zur Zeichenebene - nicht die Füllgeschwindigkeit des Kanals (3) überschreitet, um das unerwünschte seitliche Vorschiessen der Flüssigkeitsfront zu verhindern. Anzumerken ist hierbei, dass beim Darstellungsbeispiel die Höhe H der Ausnehmung (5) etwa nur der Höhe H des Kanals (3) entspricht. Vorzugsweise ist diese jedoch größer.
Die vorschlagsgemäße Vorrichtung (1) ist für verschiedenste Tests, Untersuchungen o. dgl. geeignet. Insbesondere gestattet sie immunologische oder biochemische Test, beispielsweise von Blut (19), Blutplasma o. dgl.
Gemäß einer Ausführungsvariante kann der Kanal (3) mehrere Untersuchungsbereiche bzw. Sammelbereiche (20) bilden, die nacheinander mit der Probenflüssigkeit (2) füllbar sind. So ist es beispielsweise möglich, verschiedene Untersuchungen nacheinander durchzuführen und/oder die Probenflüssigkeit (2) nacheinander verschiedenen Reagenzien, insbesondere Trockenchemikalien, die nacheinander gelöst werden, auszusetzen.
Gemäß einer anderen Ausführungsvariante kann sich an einen ersten Untersuchungs- oder Sammelbereich (20) ein zweiter Untersuchungs- oder Sammelbereich (20) anschließen, wobei der zweite Bereich vorzugsweise eine wesentlich höhere Kapillarität, beispielsweise durch ein eingesetztes Vlies o. dgl., aufweist. So kann die Probenflüssigkeit (2) nach Füllen des ersten Bereichs und insbesondere nach Lösen einer bedarfsweise dort vorhandenen Trockenchemikalie anschließend in den zweiten Bereich gesaugt bzw. geleitet werden, wobei die Trockenchemikalie aus dem ersten Bereich ausgewaschen und so beispielsweise eine weitere Untersuchung im ersten und/oder zweiten Bereich ermöglicht wird.
Gemäß einer weiteren Ausführungsvariante sind eine erste Chemikalie, insbesondere eine Trockenchemikalie, vorzugsweise in der Zuführeinrichtung (13) oder Trenneinrichtung (15), und mindestens eine zweite Chemikalie, insbesondere eine Trockenchemikalie, vorzugsweise im Kanal (3) bzw. Sammelbereich (20), vorgesehen. Dies gestattet eine effektive Manipulation bzw. Beeinflussung der Probenflüssigkeit (2), des Bluts (19) o. dgl. Vorzugsweise ist zur Untersuchung von Blutplasma die erste Chemikalie derart ausgebildet, dass diese eine Gerinnung des Bluts (19) verhindert bzw. verzögert. Hierzu kann beispielsweise zur Herstellung von EDTA-Blut als erste Chemikalie EDTA (Ethylene Diamine Tetraacetic Acid (Äthylendiamintetraessigsäure)) verwendet werden. Dabei bindet das EDTA das Calcium des Bluts, das als Faktor IV für die Blutgerinnung erforderlich ist. Anschließend dient die zweite Chemikalie, vorzugsweise ein Chemikaliengemisch, einer Untersuchung bzw. einer Bestimmung eines oder mehrerer Parameter im Blutplasma, wie Glukose, Ketone oder Laktat.
Vorzugsweise ist zur Untersuchung mindestens eines intrazellulären Parameters, wie des Hämoglobin-Werts oder des Calcium-Werts in Blut (19), die erste Chemikalie derart ausgebildet, dass sie Zellen, wie Blutzellen, lysiert und das Calcium o. dgl. freisetzt. Hierzu wird beispielsweise Lysinpuffer eingesetzt.
Anschließend dient die zweite Chemikalie, vorzugsweise ein Chemiekaliengemisch, einer Untersuchung bzw. Bestimmung des Parameters, insbesondere des Calcium-Gehalts. Ein Bestandteil des Gemisches, vorzugsweise der Chelatbildner 8-hydroxychinolin, wird dazu eingesetzt, die Reaktion störende Magnesiumionen aus der Reaktion zu entfernen. Ein anderer Komplexbildner, vorzugsweise o-Kresolphthalein, bildet mit Calcium unter alkalischen Bedingungen einen farbigen Komplex.
Die Extinktion des Farbkomplexes ist bei einer Wellenlänge von 570 nm proportional zur Calcium-Konzentration. Sie wird direkt im Kanal (3) bzw. Sammelbereich (20) oder ggf. nach Entnahme bestimmt. Jedoch sind auch andere Bestimmungen oder Verfahrensweisen möglich. Insbesondere kann die Extinktion auch bei anderen Wellenlängen und/oder zur Bestimmung anderer Komplexe, Parameter o. dgl. eingesetzt werden. Entsprechendes gilt für sonstige, vorzugsweise optische Bestimmungsverfahren, wie Fluoreszenzmessungen o. dgl.
Gemäß noch einer weiteren Ausführungsvariante ist in der Abdeckung (7) und/oder im Träger (6) eine nicht dargestellte Entnahmeöffnung vorgesehen, um eine Entnahme der Probenflüssigkeit (2), insbesondere von abgetrenntem Blutplasma o. dgl., zu ermöglichen. Die Entnahmeöffnung steht vorzugsweise mit einem zumindest relativ großvolumigen Speicherbereich (20) o. dgl. des Kanals (3) in Verbindung, um ein gewünschtes bzw. ausreichendes Entnahmevolumen bereitstellen zu können.

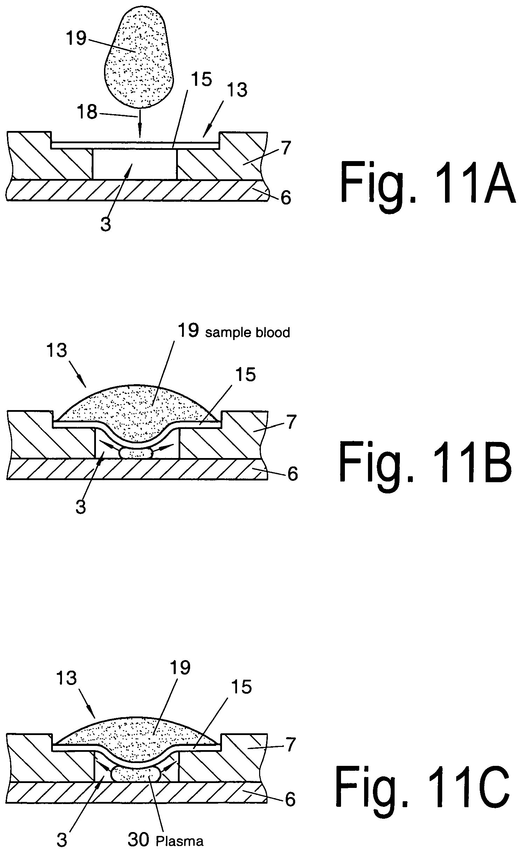

In der Regel ist der Abstand der Membranfläche zum Kanalboden gleich der Kanalhöhe wie Fig. 11A darstellt. Dabei stellt sich das Problem, dass das Trennelement zur Blutseperation eine fluidische Barriere für den ungehinderten Fluss des Plasmas in den Kanal darstellen kann. Dies ist dadurch bedingt, dass als Trennelement eine Membran oder ein Filterelement eingesetzt wird, wobei die Membran oder das Filterelement aus einem verflochtenen Fasernetzwerk oder aus einem porösen Material besteht. Als Materialien sind zu einem Vlies verbundene oder komprimierte Kunstfasern oder auch poröse Keramiken wie auch Metallnetze verwendbar. Das Filtermaterial weist durch die Netzstruktur dünne verzweigte Kanäle mit hoher Kapillarkraft auf, wodurch fluidische Bestandteile im Filter oder der Membran gehalten werden. Die Membran weist eine Porengröße von 0,01 Mikrometern bis zu 1,2 Mikrometern, insbesondere 0,2 bis 0,6 Mikrometer auf. Die Membrandichte beträgt 50 Mikrometer bis 500 Mikrometer, bevorzugt mit 120 µm bis 180 µm. Die Porosität, also der nicht mit Material ausgeführte Volumenanteil der Membran beträgt 40-90 % bevorzugt 70 bis 80 %. Als Porenmaterial können verschiedene Materialien, wie Nylon, insbesondere isotrop geschäumtes Nylon 60, mit einem Porenvolumen größer 70%, und einer Porengröße von 0,45 Mikrometern oder auch bevorzugt hydrophiles Polyvinyldifluorid mit einer Porengröße von 0,6 Mikrometern.
Wird nun ein Tropfen Blut in den Einlassbereich in Zuführeinrichtung (18) eingebracht, so legt sich ein halbkugelförmiger Tropfen Blut auf die Oberfläche der Membran (15) wie in Fig.11b zu beobachten ist. Durch die Schwerkraft und den Fluiddruck fließt das Blutplasma unter Zurückhaltung der größeren Blutpartikel durch die Kanäle der Membran (15) und bildet durch den hydraulischen Druck an der Unterseite einen Plasmafilm oder Plasmatropfen aus, der an der Membran anhaftet. Die kleinen Blut- bzw. Plasmamengen oder auch insbesondere bei großen zu füllenden Totvolumen zwischen der Membran und dem Kanalboden kann die fluidische Anbindung zwischen Plasma und Kanal ausbleiben. Oft fließt zwar ein Plasmastrom insbesondere an den Kanalwänden bis
zum Kanalboden und füllt den Kanal (3) oder den Sammelbereich (20) langsam. Der Start des Befüllvorganges wird dadurch aber verzögert, was ungewollt lange Fließzeiten bedingt, die sich negativ auf die Funktion einer an die fluidische Kanalstruktur angeschlossenen Diagnose- oder Analysevorrichtung auswirken.
Das Totvolumen im Befüllbereich zwischen dem Trennelement und dem Kanal wirkt dabei also wie eine Impedanz oder ein Widerstand für die Flussrate des Plasmas.

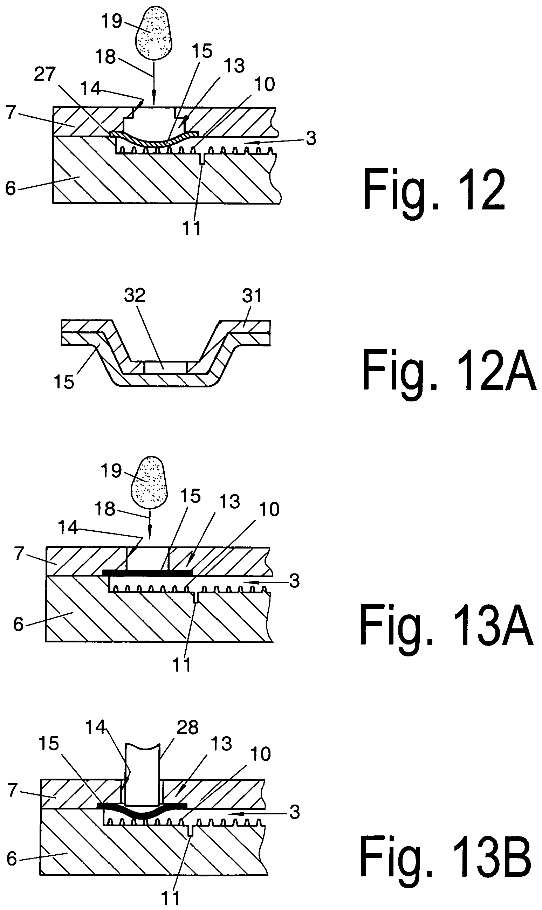

Eine weitere Aufgabenlösung der vorliegenden Erfindung ist es, diese Impedanz gezielt einzustellen, insbesondere auf ein Mindestmaß zu verringern.
Vorteilhaft kann der Fließwiderstand durch eine Ausführung des Trennelements (15) gemäß Fig. 11B minimiert werden. Dazu ist das Trennelement (15) in Richtung des Kanalbodens konvex ausgeformt, so dass es bevorzugt in einem Zentralbereich auf dem Kanalboden aufliegt oder alternativ der Apex der konvexen Ausformung nahe an den Kanalboden heranreicht. Der Abstand des Trennelements, vom Boden der Fülleinrichtung, insbesondere vom Kanalboden beträgt dabei vorzugsweise 1 Mikrometer bis 100 Mikrometer, insbesondere 10 Mikrometer bis 25 Mikrometer.
Hierdurch wird erreicht, dass die durch die Schwerkraft oder den Hydraulischen Druck aus der Unterseite der Membran austretende Plasmaflüssigkeit den Kanal direkt benetzt und ausgehend von diesem Benetzungspunkt in den Kanal einströmt, wie die Fig. 11B und die Figur 11C schematisch darstellen.
In einer bevorzugten Ausführung beträgt der Durchmesser der verwendeten Membran (15) 2 bis 10 Millimeter, insbesondere 250 bis 350 Mikrometer.
Vorteilhaft liegt der Höhenwert W der Wölbung oder des Apexes, wie in Figur 12 a schematisch gekennzeichnet im Bereich der Membrandichte. Im vorbezeichneten Beispiel beträgt bei einer Membrandicke von 250 Mikrometern der Wert W bevorzugt 100 Mikrometer bis 300 Mikrometer. Der Höhenwert der Wölbung soll vorteilhaft in etwa die Höhe eines in den Einlassbereich Kanals oder einer unter der führenden Membran (15) liegenden Kammer entsprechen. Da die Kanaltiefe zu Figur 1 ausgeführt bevorzugt 50-200 Mikrometer beträgt, kann die Höhe W der Wölbung auch angepasst an die Grabentiefe im Bereich von 50 bis 200 Mikrometern variieren.
Vorteilhaft weisen die Kanalwände und insbesondere der Kanalboden den Volumenfluss Kapillarverstärkende Elemente (10) auf, wie sie in Fig. 2 dargestellt. Besonders vorteilhaft wird ein Element (17) mit vertikalen Kerben am Kanalboden eingesetzt, wie es in der AP 101 3341 B1 offenbart ist. Durch die Kerbgeometrie wird ein vertikaler Strom vom Einfüllbereich durch das Trennelement zum Kanalboden initiiert und unterstützt.

Fig. 12 zeigt solche Elemente am Kanalboden, wobei eine Vielzahl von Elementen derart zueinander am Boden des Kanals angeordnet sind, dass bedingt durch die Kapillarwindung der Zwischenräume ein horizontaler Volumenstrom des Fluides oder Plasmas in einer Sammelkammer (20) in Kanalrichtung erfolgt.
Vorteilhaft wird die konvexe Wölbung der Membran dadurch erreicht, dass die Membran bei ihrer Befestigung in Richtung ihres Zentrums gestaucht wird, wodurch diese sich zum Zentrum hin durchbiegt. Dies kann dadurch erreicht werden, dass der Durchmesser der Membran größer gewählt wird als der Durchmesser des Raumes in welchem die Membran befestigt, insbesondere verklebt wird. Mit einem entsprechenden Haltewerkzeug (nicht dargestellt), dass eine konvexe Oberflächenform aufweist wird die Membran in den Befestigungsbereich eingeführt und verklebt. Durch die konvexe Formung des Werkzeuges erfolgt dabei die Ausformung der Membrandurchbiegung.

Alternativ zur Verklebung können auch thermische Verfahren, wie das Schweißen, insbesondere Ultraschallverschweißen zur Befestigung der Membran eingesetzt werden, wobei die Membran vorteilhaft auch hier mit einem vorgeformten Haltewerkzeug zwischen zwei Kunststoffelementen der Vorrichtung eingepresst wird.

Alternativ zum Ausformen des Trennelements, der Membran während des Befestigungsvorganges, kann die konvexe Durchbiegung des Trennelements auch vorab durch Prägen der Form in das Trennelement erfolgen.
Bei metallischen Filterelementen ist beispielsweise denkbar, diese in eine gewölbte, insbesondere konvexe Form zu pressen oder zu biegen.
Bei Fliesmaterialien wäre ein Prägevorgang in einem entsprechen geformten Werkzeug unter Ausübung von Druck und/oder Temperatur und/oder
zusätzlich eingebrachten chemischen Fixiermitteln bzw. Klebemitteln denkbar. Alternativ könnte schon bei der Vliesherstellung aus Kunstfasern vorteilhaft vorgesehen werden beim Nadeln und Verfestigen des Vliesgewebes eine konvexe Form einzuprägen.

In einer weiteren vorteilhaften Ausführung ist das Trennelement zwei- oder mehrteilig, insbesondere zweischichtig, aufgebaut, wobei eine flexible Membran an einem festen Haltelement, insbesondere Membranhalter (31) angeordnet ist, wie Fig. 12 A zeigt. Vorteilhaft wird die Membran im Außenbereich des trichterförmigen Halteelements verklebt, sie kann aber auch durch Klemmelemente gehalten werden. Der trichterförmige Membranhalter oder Formeinsatz (29) weist in einem zentralen Bereich eine Durchtrittsöffnung oder Bohrung (32) auf, so dass bei Befüllen des Trichters mit Blut dieses durch die Öffnung (32) in die Membran eintreten kann.

Bei einer weiteren vorteilhaften Ausführung der Erfindung gemäß Fig. 13 ist als Trenneinrichtung (15) eine Membran (15) vorgesehen. Bei Zugabe eines Bluttropfens (19) in den Zuführungsbereich (18) und die Öffnung (14) der Abdeckung (6) legt sich der Tropfen auf die in Fig. 13A ebene Membran (15).
Im folgenden Schritt wird, wie der Fig. 13B zu entnehmen ist, ein Stößel (28) in die Öffnung (14) eingeführt, wobei der Stößel die Membranfläche in Richtung zum Kanalinneren verformt, so dass sich eine konvexe Membranform einstellt.

Der Stempel (28) ist an seinem die Membran kontaktierenden Ende ebenfalls bevorzugt gewölbt.

Das Einführen des Stößels (28) kann sowohl manuell durch eine Bedienperson erfolgen oder durch eine automatisierte Bedienvorrichtung mit aktuatorischen Antriebsmitteln. Im letzteren Fall ist der Stößel (28) an einem Stellantrieb angebracht, wobei der Stellantrieb den Stößel derart bewegt, dass dieser die Membran zum Kanalboden hinunterdrückt. Der Stellantrieb kann durch piezo-elektrische Stellglieder oder einen Schrittmotor oder andere geeignete mechanische oder elektrische Stellglieder erfolgen. Bevorzugt erfolgt das Niederfahren des Stößels (28) in Abhängigkeit von dem zu durchlaufenden Analyseschritt.

An der Vorrichtung können zur automatisierten Bewegung des Stößels (28) Sensoren angebracht sein, die das Zuführen eines Bluttropfens (19) erfassen und den Stößel oder Stempel (28) über ein Steuergerät, insbesondere einen Mikroprozessor, der die Sensorsignale aufnimmt und verarbeitet, ansteuern.

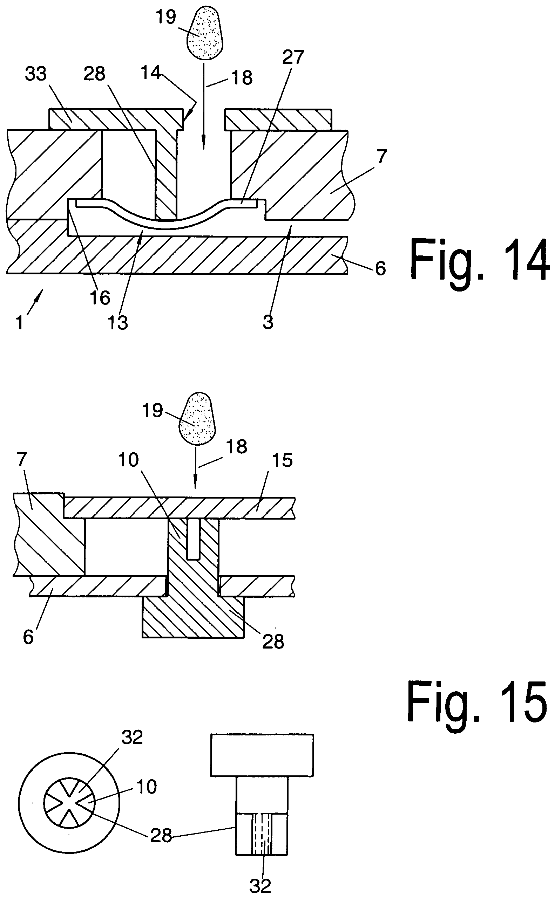

In einer anderen Ausgestaltung der Erfindung gemäß Fig. 14 wird der Kanal (3) durch eine Ausnehmung (5) in Träger (6) und eine deckungsgleiche Aussparung in der Abdeckung (7) gebildet. Die Abdeckung (7) weist im Endbereich des Kanals (3) eine Öffnung auf. Diese Öffnung (14) wird zur Oberseite der Abdeckung (7) von einem Druckelement (33) abgeschlossen.

Das Deckelelement (33) umfasst einen Stempel (28) und eine Öffnung (14) durch die ein Bluttropfen (19) dem Zuführbereich (18) zugeführt werden kann. In der Aussparung (16) der Abdeckung ist ein Trennelement (15), insbesondere eine Filtermembran angeordnet und befestigt.
Die Befestigung kann beispielsweise durch Verkleben oder Verschweißen mit der Abdeckung (7) hergestellt werden. Bei der Herstellung der Vorrichtung (1) gemäß Fig. 14 wird in einem ersten Schritt die Befestigung der Membran an der Abdeckung (7) durchgeführt. In einem nachfolgenden Herstellungsschritt werden die mit der Membran versehende Abdeckung (7) und der Träger (6) miteinander verbunden.

In einem weiteren ersten Schritt nachfolgenden Herstellungsschritt wird das Deckelelement (33) an der Abdeckung (7) befestigt, wodurch der Stößel (28) die Membran konvex verformt, sodass das Totvolumen in der Zuführeinrichtung (13) verringert wird und der Apex der konvexen Membran (27) bis in die Nähe des Kanalbodens gelangt. Hierdurch wird eine Vorrichtung (1) erreicht, bei der ein deutlich verringerter Fluidwiderstand zwischen der Membran (27) und dem Kanal (3) vorliegt.
In einer Ausführung der Erfindung gemäß Figur 15 wird der Stempel (28) in eine Bohrung im Träger (6) in den Zuführbereich eingesetzt. Hierzu weist der Stempelschaft einen an den Stempelkopf grenzenden ersten Abschnitt auf, der in seiner Länge der Dicke des Trägers (6) im Bereich der Bohrung entspricht und durch das Einsetzen des Stempels die Bohrung dichtend verschließt.

Ein zweiter Abschnitt des Stempelschaftes ist mit Kerben oder einer Profilierung versehen oder weist durchgehende Durchbrechungen in Schaftlängsrichtungen auf. Dieser zweite Abschnitt des Stempelschaftes erstreckt sich vom Boden des Zuführungsbereiches (18), insbesondere dem Boden des Kanals (3) oder dem Boden einer Sammelkammer (20) bis zum Trennelement (15) und kontaktiert dieses, so dass durch den profilierten Stempelschaft eine vertikale Fluidverbindung zwischen dem Boden und einer Membran (15) aufgebaut wird.

Besonders vorteilhaft kann der Stempelschaft mit Erhöhungen bzw. Leitelementen (10) an seiner bevorzugt zylindrischen Mantelfläche versehen sein, die einen Kapillarfluss des abgetrennten Plasmas unterstützen.

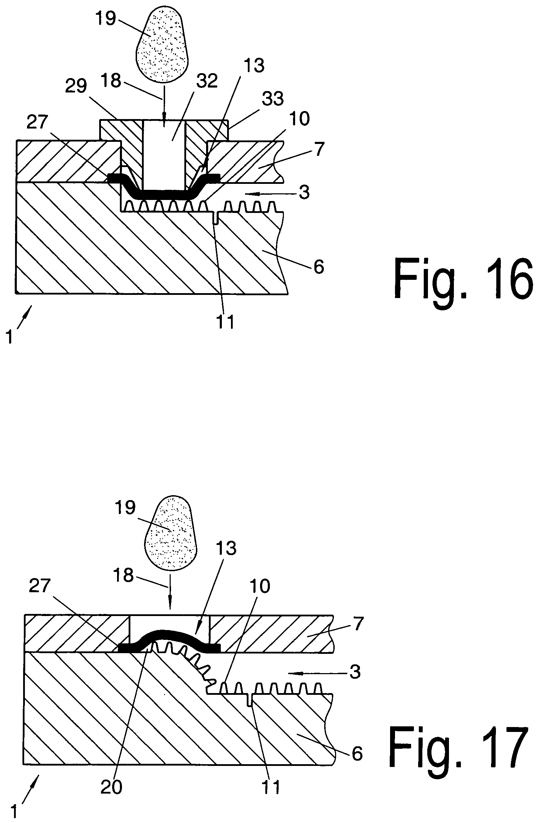

Fig. 16 zeigt eine Vorrichtung, bei der das Deckelelement (33) mit einer zentralen Bohrung (32) versehen ist, durch die ein Bluttropfen (19) in den Zuführungsbereich (18) eingebracht wird.
Das Deckelelement (33) ist an der Abdeckung (7) befestigt, beispielsweise mittels Ultraschall verschweißt.

Druckelement (33), Abdeckung (7) und Träger (6) sind bevorzugt aus Kunststoffmaterial.

Die Membran (27) ist zwischen der Abdeckung (7) und dem Träger (6) eingeklemmt, insbesondere verschweißt.
In der Ausführungsform nach Figur 16 ist das Druckelement (33) als Formeinsatz (29) gestaltet, wobei der Formeinsatz (29) in Richtung des Kanals (3) derart dreidimensional ausgestaltet ist, das die Membran konvex verbogen wird und im fluidischen Kontakt zum Boden des Kanal (3), insbesondere zu den Leitelementen (10) am Boden des Kanals kommt.

Bei einer Ausgestaltung nach Fig. 17 wird bei der Herstellung der Vorrichtung (1) das Trennelement (15) zunächst mit der Abdeckung (7) verbunden und schließt den

Zuführbereich (18) der Zuführungseinrichtung (13) nach unten zum Eintrittsbereich des Kanals hin ab.

Das Trägerelement (6), dass eine Aussparung in Form eines Kanals (3) aufweist, ist im Bereich der Öffnung der Abdeckung (7), also im Bereich der eben an die Abdeckung (7) angebrachten Membran (27) konstruktiv so gestaltet, dass in einem zum zentralen Bereich der Öffnung gegenüberliegend angeordneten Bereich des Trägers (6) die Trägeroberfläche über die Verbindungsebene zwischen Träger (6) und Abdeckung (7) hinaus ausgeformt ist. Dies kann beispielsweise dadurch erreicht werden, dass wie in Fig. 17 vorgesehen sich die Oberfläche des Trägers (6) in diesen Bereich hinein konvex auswölbt, diese gegen die Membran (27) drückt und entsprechend der Form des Trägers (6) entsprechend auslenkt. Vorteilhaft ist die Trägeroberfläche auch in diesem Bereich mit Leitelementen (10) versehen, die eine horizontale Fluidströmung des Plasmas bedingen.

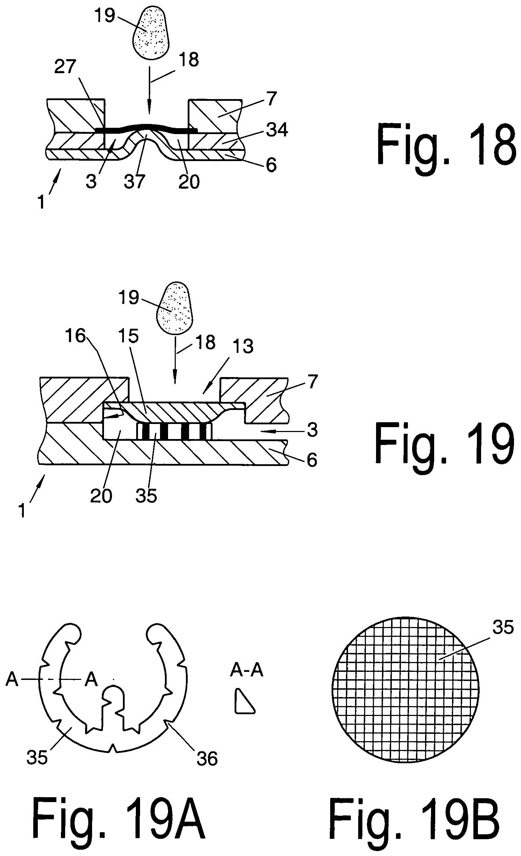

Bei der Ausführungsform gemäß Fig. 18 ist die Vorrichtung (1) dreilagig aufgebaut. Hierbei wird der Träger (6) über ein Zwischenelement (34), in diesem Fall das den Kanal bildende Kanalelement (34) mit der Abdeckung verbunden. Das Zwischenelement (34) wird dazu beispielsweise mit dem Träger (6) und mit der Abdeckung (7) verklebt. Das Zwischenelement (34) ist bevorzugt eine beidseitige Adhesivfolie. Die Kanalstrukturen, insbesondere der Kanal (3), sind als Aussparungen (16) in das Zwischenelement eingebracht, beispielsweise durch Ausstanzen aus der fertigen Form oder als Aussparungen beim Form- oder Gießprozess.

Vorteilhaft können in dieser Ausführungsform, in welcher alle Leitkanäle und Fluidkammern im Zwischenelement angeordnet sind, die Abdeckung (7) und der Träger (6) als ebene Elemente ohne Ausnehmungen (5) für die fluidleitenden Strukturen ausgeführt werden, was den Einsatz von präzisen kostenintensiven Mikroformwerkzeugen deutlich reduziert und die Herstellung einfacher macht.

Bei der Ausführungsform nach Fig. 18 ist zur Herstellung eines fluidischen Kontaktes und zur Verminderung des Flusswiderstandes im Einlassbereich des Kanals vorgesehen, das Trägerelement (6) in Richtung der Membran (27) auszuformen, insbesondere mindestens einen Zapfen (37) in das Trägerelement (6) einzubringen der aus der Verbindungsebene zwischen dem Kanalelement (34) und dem Trägerelement (6) in Richtung der Membran herausragt und einen fluidischen Kontakt zur Membran herstellt.

Der Zapfen (37) kann beispielsweise bei der Herstellung des Trägers (6) direkt durch Abformen oder nachgeordnet durch mechanisches und/oder thermisches Einprägen in das Trägerelement eingebracht werden.

In einer Ausführungsform gemäß Fig. 19 ist der Kanal (3) als Ausnehmung (5) in den Trägern (6) eingeformt.
Die Trennvorrichtung (15), in diesem Fall ein Filterelement (15), ist in einer Aussparung (16) der Abdeckung (7) angeordnet und bildet zusammen mit dem Einlegeelement oder Einsatz (35) die Zugführungseinrichtung (13). Wird nun ein Tropfen (19) Blut dem Zuführbereich (18) zugeführt, so wird das Blut von dem Filter (15) aufgesaugt und gefiltert, wobei das in Kanalrichtung austretende Plasma von dem an den Filter (15) kanalseitig angeordnetem Einsatz (35) aufgenommen wird.

Der Einsatz (35) ist geometrisch derart ausgestaltet, dass seine Höhe in etwa der Höhe des Zwischenraumes zwischen Kanalboden und der Unterseite des Filters (15) entspricht und der Einsatz (35) dabei sowohl den Kanalboden oder Kammerboden im Einlassbereich wie auch die Unterseite des Filters kontaktiert. Bevorzugt ist der Einsatz (35) ein Einlegeelement (35), was bedeutet, dass der Einsatz (35) beim Verbinden des Trägers (6) mit der Abdeckung (7) seine Befestigung dadurch erfährt, dass er vom Anpressdruck zwischen dem Filter und dem Träger (6) gehalten wird.

Der Einsatz kann beispielsweise als O-Ring aus einem elastischen Kunststoff oder aus einem Gummi gefertigt werden.

In einer bevorzugten Ausführung des Einsatzes (35) nach Fig. 19B besteht dieser aus einem weiteren Filtermaterial, dies kann eine poröse Keramik, ein Schwamm aus Fasermaterial, ein metallisches Gitter- oder Maschenelement oder ein sonstiges geeignetes Element aus kanaltragenden Strukturen sein.

Als Materialien sind auch gelartige Schwämme oder Polymere, wie Polysacharide oder Silikone einsetzbar. Beispiele für derartige Polymere sind Saccharose, Polyarylamid oder Agarose.
Vorteilhaft können in das schwamm-oder gelartige Material Reagenzien eingebracht sein, wie beispielsweise Anticoagulanten (K2EDTA).
In einer anderen bevorzugten Ausführungsform nach Fig. 19A ist der Einsatz (35) als hufeisenförmiges Einlegeelement (35) gestaltet. Das Einlegeelement kann aus porenfreiem Kunststoffmaterial bestehen, es ist aber auch denkbar, das Einlegeelement aus einem der oben genannten kanaltragenden Materialien herzustellen.

Besonders bevorzugt weist das Einlegeelement im Randbereich mindestens eine Kerbe (36), insbesondere eine Vielzahl von Kerben (36) auf, die eine vertikale Ableitung des Plasmas in die Plasmakammer (20) oder den Kanal (3) unterstützen. Vorteilhaft ist weiterhin vorgesehen, dass der Querschnitt der Einlegeteile keilförmig ist, wie der Schnitt A-A in Fig. 19A zeigt, wobei die Spitze des Keils die Membranfläche kontaktiert und dadurch den Fluidkontakt herstellt.

Die im Volumen des Zuführbereichs vorliegende Luft kann bei der Befüllung mittels eines Bluttropfens in den Befüllbereich eingeschlossen werden.

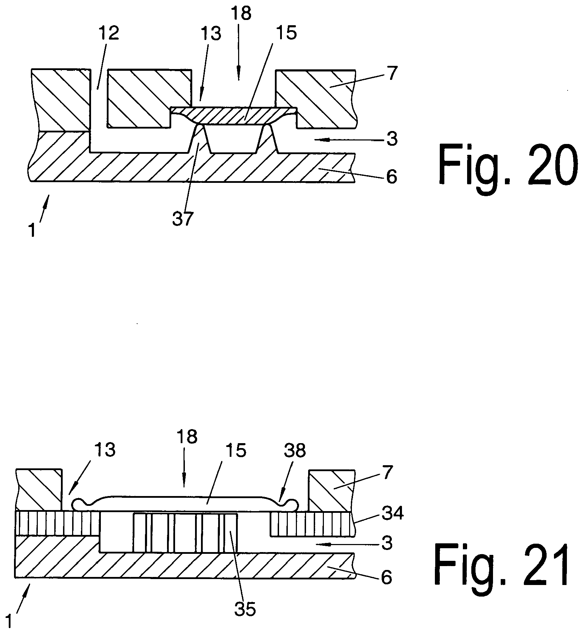

Dies kann einerseits den Effekt haben, das Luft aus dem Bereich unterhalb des Filters (15), der auf dem Zapfen (37) gemäß Figur 20 aufliegt, in den Kanal gedrückt wird. Diese Luftbläschen stellen einen großen Flusswiderstand dar und sind deshalb unerwünscht. Weiterhin kann es auch zu einem Luftstau kommen, der einen Gegendruck zum hydraulischen Druck des Plasmas aufbaut und eine große Strömungsinpedanz darstellt. Vorteilhaft ist daher vorgesehen, eine seitliche Entlüftung (12) des Sammelraumes (20) quer zum Kanal (3) anzuordnen. Eine derartige Entlüftung an der Zuführeinrichtung (13) kann bei allen Ausführungsformen gemäß den Figuren 1 bis 23 vorgesehen sein.

Um zu gewährleisten, dass bei einem Aufbau mit einem Träger (6), einer Abdeckung (7) und einem Zwischenelement (34) eine dichte Versiegelung des Filters (15) erreicht wird, wird dieser in seinem Befestigungsbereich mit einer Pressung (38) versehen, die das Filtermaterial im Bereich der Pressung (38) komprimiert. Bei einer derartigen Vorrichtung nach Fig. 21 ist in einem Trägeelement (6) eine Ausnehmung (5) eingeformt, die den Kanal (3) bildet. Bei dem Zwischenelement handelt es sich um ein auf beiden Seiten mit einem Adhäsiv versehenen folienartiges Kunststoffteil, wobei das Ädhäsiv den Kontakt durch Verklebung sowohl zur Abdeckung (7) als auch zum Träger (6) herstellt und diese miteinander verbindet. Kontaktierend zum Filter ist ein Einsatz (35) in Form eines Einlegeteiles (35) im Bereich der Zuführungseinrichtung angeordnet.

Bei einer Ausführungsform gemäß Fig. 18 ist die Trenneinrichtung oder der Filter (15) in die Abdeckung (7) eingeklebt oder eingeschweißt, wobei das Schweißen beispielsweise mittels Ultraschall oder mittels thermischem Verschweißen erfolgt.

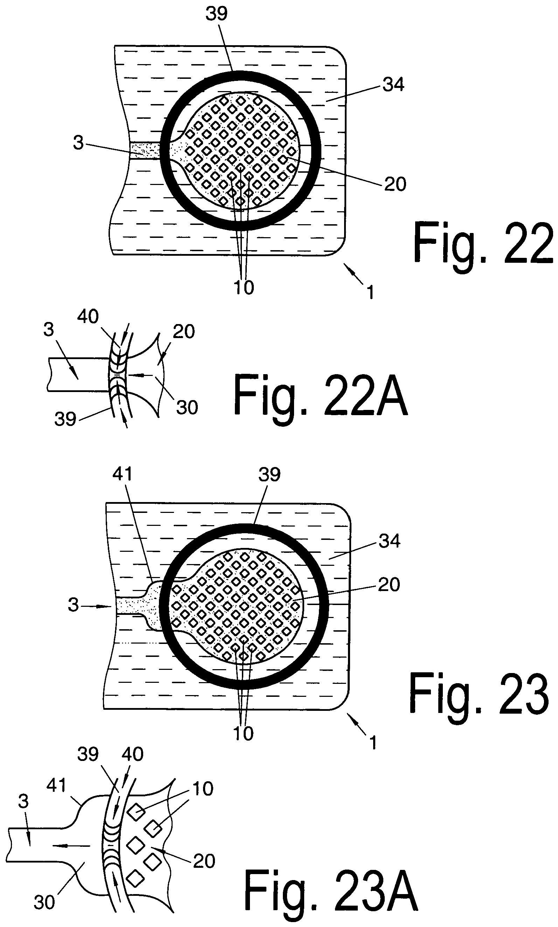

In einer Ausführung dieser Anordnung nach Fig. 22 ist der Zuführbereich von oben dargestellt. Hierbei ist in der Draufsicht das Zwischenelement (34) zu sehen, das beispielsweise ein Kanalelement mit Ausstanzungen ist, die eine Probensammelkammer (20) im Zuführbereich und einen Kanal (3) bilden. Von dem darunter liegenden Träger (6) sind im Bereich der Probenkammer (20) die Leitelemente (10) zu sehen.

Über der Ebene des Zwischenelementes, ist die Schweißlinie (Befestigungslinie) (39) schematisch abgebildet, die in der oberen Abdeckung (7) liegend den Filter oder die Filtermembran (15) mit der Abdeckung verhindert. Das Zwischenelement ist insbesondere eine Folie, die auf beiden Seiten mit einem Klebemittel versehen ist.

Beim Einfüllen eines Tropfen Blutes fließt das abgetrennte Plasma in die Sammelkammer (20) und wird von den Leitelementen unterstützend in den Kanal (3) abgeleitet. Der Einlassbereich des Kanals (3) stellt dabei eine deutliche und abrupte Verringerung des Fließquerschnittes dar.

Wie die Fig. 22A zeigt, kann es dabei zum Einströmen von Luft in den Kanal (3) kommen, wodurch strömende Luftblasen mit in den Kanal gelangen können, die den Strömungswiderstand deutlich erhöhen oder den Strömungsfluss gänzlich zum Erliegen bringen können. Insbesondere im Bereich der Befestigungslinie (39) kann es zum Quereinströmen von Luft (40) kommen, da das Filtermaterial durch die Komprimierung an dieser Stelle beim Schweißen einen Hohlraum hinterlässt, aus dem Luft einströmen kann.

In einer vorteilhaften Ausgestaltung des Überganges von der Probenkammer (20) zum Kanal (3) ist deshalb gemäß Fig. 23 vorgesehen, den Flussquerschnitt von der Sammelkammer zum Kanal entlang eines Übergangsbereiches kontinuierlich zu verringern. Dies kann beispielsweise stufenweise erfolgen, indem wie aus Figur 22 ersichtlich, zunächst eine Verringerung des Querschnittsbereichs soweit erfolgt, das der verringerte Querschnitt (41) etwa das 2 bis 5 fache des Querschnitts des Kanals (3) beträgt.

Wie Figur 23A aufzeigt ist die Querschnittsstufe (41) so angeordnet, dass die umlaufende Befestigungslinie (39) den Auslassbereich aus der Sammelkammer (20) im Bereich der Querschnittsstufe bremst und keine Kreuzung zum Kanal (3) bildet, wodurch ein direktes Einströmen von Luft (40) in den Kanal (3) vermieden wird.

Zwar strömt auch in diesen Ausführungsstrom Luft in den Bereich der Querschnittsstufe (41) ein, da diese aber einen größeren Querschnitt aufweist, dauert es entsprechend länger bis eingeströmte Luftblasen diesen Querschnitt blockieren und zum Abreißen des Fluidstroms führen können.

In einer Vielzahl der dargestellten Ausführungsbeispiele weist der Boden des Kanals (3) oder der Boden der Zuführeinrichtung (18) Leitelemente (10) auf. Diese Leitelemente tragen zur Unterstützung der Benetzung durch einen vertikalen Fluidfluss bei. Bei geeigneter Positionierung zueinander wird durch die kapillar wirkenden Zwischenräume zwischen den Leitelementen (10) zudem ein horizontaler Fluidfluss unterstützt.
Alle diesen Ausführungsformen ist gemein, dass die Leitelemente kein wesentlicher Funktionsbestandteil sind. In gleicher Weise wie ein Leitelement (10) wirkt auch der jeweils vorhandene Kapillarspalt zwischen einem Filter und oder einer Membran (15) und dem Boden eines Kanals (3) oder einer Zuführeinrichtung (18), da sich durch die Wölbung des Bodens zur Membran an den Berührungsflächen bzw. den Nährungsflächen keilförmige Kapillarspalte mit geringer Höhe und hoher Kapillarität einstellen lassen.

## Patentansprüche

1. Vorrichtung (1) zur Aufnahme von Blut (19) und Abtrennung von Blutbestandteilen, wie Blutplasma, als Probenflüssigkeit (2), mit einer Zuführeinrichtung (13) zur Aufnahme des Bluts (2) und einer Membran (15) zur Abtrennung von Blutbestandteilen als Probenflüssigkeit (2), einen die Probenflüssigkeit (2) durch Kapillarkräfte aufnehmenden Kanal (3) und einer Fülleinrichtung zum Füllen des Kanals (3) mit Probenflüssigkeit (2) in einem Einlass- oder Zuführbereich (18) des Kanals (3), **dadurch gekennzeichnet, dass** die Membran (15) konvex geformt ist und mit dem Apex der Ausformung in die Fülleinrichtung in Füllrichtung hineinragt und dass die Membran (15) in einem Zentralbereich auf dem Boden der Fülleinrichtung aufliegt, oder dass der Abstand des Apex der Membran (15), vom Boden der Fülleinrichtung 10 Mikrometer bis 25 Mikrometer beträgt, so dass aus der Unterseite der Membran austretende Probenflüssigkeit den Boden direkt benetzt.

2. Vorrichtung nach Anspruch 1, **dadurch gekennzeichnet, dass** der Boden der Fülleinrichtung der Boden des Kanals (3) und/oder der Boden einer Sammelkammer (20) ist.

3. Vorrichtung nach Anspruch 1, **dadurch gekennzeichnet, dass** die Membran (15) vor dem Einsetzen in die Vorrichtung mittels Prägen in die konvexe Form gebracht wird.

4. Vorrichtung nach Anspruch 1, **dadurch gekennzeichnet, dass** die Membran (15) an einem trichterförmigen Halteelement (31) befestigt ist.

5. Vorrichtung nach Anspruch 4, **dadurch gekennzeichnet, dass** das Halteelement (31) in einem zentralen Bereich eine Öffnung (32) aufweist.

6. Vorrichtung nach Anspruch 1, **dadurch gekennzeichnet, dass** die konvexe Form mittels eines beweglichen Stempels oder Stößels (28) erzeugt wird.

7. Vorrichtung nach Anspruch 6, **dadurch gekennzeichnet, dass** der Stempel mittels eines Stellgliedes bewegbar ist, wobei durch Aktuierung des Stellgliedes der Plasmafluss in Gang gesetzt wird.

8. Vorrichtung nach einem der Ansprüche 6 oder 7, **dadurch gekennzeichnet, dass** der Stempel (28) an seinem die Membran kontaktierenden Ende gewölbt ist.

9. Vorrichtung nach Anspruch 1, **dadurch gekennzeichnet, dass** die Vorrichtung (1) einen Träger (6) und eine Abdeckung (7) aufweist, zwischen oder von denen der Kanal (3) gebildet sind.

10. Vorrichtung nach Anspruch 9, **dadurch gekennzeichnet, dass** die Vorrichtung ein Deckelelement (33) umfasst, dass einen Stempel (28) aufweist.

11. Vorrichtung nach Anspruch 10, **dadurch gekennzeichnet, dass** das Deckelelement (33) bei der Herstellung der Vorrichtung (1) an der Abdeckung der (7) befestigt wird.

12. Vorrichtung nach einem der vorher gehenden Ansprüche 10 oder 11, **dadurch gekennzeichnet, dass** das Deckelelement (33) seitlich des Stempels (28) eine Durchlassöffnung (14) aufweist, durch die eine Flüssigkeit in den Zuführbereich (18) eingebracht werden kann.

13. Vorrichtung nach einem der vorher gehenden Ansprüche 11 oder 12, **dadurch gekennzeichnet, dass** der Stempel (28) eine durchgehende Bohrung (32) aufweist, durch die Probenflüssigkeit (2) in den Zuführbereich (18) eingeführt werden kann.

14. Vorrichtung nach Anspruch 13, **dadurch gekennzeichnet, dass** die Seitenwandung (26) des Stempels an der Innenwandfläche Leitelemente, wie Erhöhungen (10), aufweist.

15. Vorrichtung nach einem der vorher gehenden Ansprüche 10 bis 14, **dadurch gekennzeichnet, dass** der Stempel (28) oder das Deckelelement (33) als Formeinssatz ausgestaltet sind, wobei die dreidimensionale Ausformung der Membran (15) ihre Form aufprägt.

## Claims

1. Apparatus (1) for taking up blood (19) and separating blood components such as blood plasma, as a sample liquid (2), comprising a feed device (13) for taking up the blood (2) and a membrane (15) for separating blood components as the sample liquid (2), a channel (3) which takes up the sample liquid (2) by capillary forces and a fill device for filling the channel (3) with sample liquid (2) in an inlet or feed region (18) of the channel (3), **characterised in that** the membrane (15) has a convex shape, and the apex of the convex shape projects into the filling device in the direction of filling, and **in that** the membrane (15) rests on the base of the fill device in a central region, or **in that** the distance of the apex of the membrane (15) from the base of the filling device is from 10 to 25 micrometres, and therefore sample liquid emerging from the lower face of the membrane wets the base directly.

2. Apparatus according to claim 1, **characterised in that** the base of the fill device is the bottom of the channel (3) and/or the bottom of a collecting chamber (20).

3. Apparatus according to claim 1, **characterised in that** the membrane (15) is brought into the convex shape by means of moulding before being inserted in the apparatus.

4. Apparatus according to claim 1, **characterised in that** the membrane (15) is attached to a funnel-shaped retaining element (31).

5. Apparatus according to claim 4, **characterised in that** the retaining element (31) comprises an opening (32) in a central region.

6. Apparatus according to claim 1, **characterised in that** the convex shape is produced by means of a movable punch or ram (28).

7. Apparatus according to claim 6, **characterised in that** the punch can be moved by means of a positioning member, the plasma flow being set in motion by actuation of the positioning member.

8. Apparatus according to either claim 6 or claim 7, **characterised in that** the punch (28) is curved at the end thereof which contacts the membrane.

9. Apparatus according to claim 1, **characterised in that** the apparatus (1) comprises a support (6) and a cover (7), between which or by which the channel (3) is formed.

10. Apparatus according to claim 9, **characterised in that** the apparatus comprises a cover element (33) which has a punch (28).

11. Apparatus according to claim 10, **characterised in that** the cover element (33) is attached to the cover (7) during the production of the apparatus (1).

12. Apparatus according to either of the preceding claims 10 or 11, **characterised in that** the cover element (33) comprises, to the side of the punch (28), an outlet opening (14) through which a liquid can be introduced into the feed region (18).

13. Apparatus according to either of the preceding claims 11 or 12, **characterised in that** the punch (28) has a through-hole (32) through which sample liquid (2) can be introduced into the feed region (18).

14. Apparatus according to claim 13, **characterised in that** the side wall (26) of the punch comprises, on the surface of the inner wall, deflector elements such as elevations (10).

15. Apparatus according to any of the preceding claims 10 to 14, **characterised in that** the punch (28) or the cover element (33) are constructed as shaping inserts, the three-dimensional shape of the membrane (15) stamping the shape thereof.

## Revendications

1. Dispositif (1) pour la réception de sang (19) et pour la séparation de composants sanguins tels que le plasma sanguin, en tant que fluide échantillon (2) comportant un système d'acheminement (13) pour la réception du sang (2) et une membrane (15) pour la séparation de composants sanguins en tant que fluide échantillon (2), un canal (3) recevant le fluide échantillon (2) par des forces capillaires et un système de remplissage pour le remplissage du canal (3) avec un fluide échantillon (2) dans une zone d'entrée ou d'acheminement (18) du canal (3), **caractérisé en ce que** la membrane (15) est formée de façon convexe et dépasse avec l'apex de la forme dans le système de remplissage dans la direction de remplissage et **en ce que** la membrane (15) repose dans une zone centrale sur le fond du système de remplissage, ou **en ce que** la distance de l'apex de la membrane (15) depuis le fond du système de remplissage est de 10 micromètres à 25 micromètres de sorte que du fluide échantillon sortant de la face inférieure de la membrane mouille directement le fond.

2. Dispositif selon la revendication 1, **caractérisé en ce que** le fond du système de remplissage est le fond du canal (3) et/ou le fond d'une chambre de collecte (20).

3. Dispositif selon la revendication 1, **caractérisé en ce que** la membrane (15) avant l'insertion dans le dispositif prend la forme convexe par estampage.

4. Dispositif selon la revendication 1, **caractérisé en ce que** la membrane (15) est fixée à un élément de retenue en forme d'entonnoir (31).

5. Dispositif selon la revendication 4, **caractérisé en ce que** l'élément de retenue (31) présente une ouverture (32) dans une zone centrale.

6. Dispositif selon la revendication 1, **caractérisé en ce que** la forme convexe est produite au moyen d'un poinçon ou d'un poussoir (28) mobile.

7. Dispositif selon la revendication 6, **caractérisé en ce que** le poinçon peut être déplacé au moyen d'un actionneur, dans lequel par actionnement de l'actionneur, le flux de plasma est mis en oeuvre.

8. Dispositif selon l'une des revendications 6 ou 7, **caractérisé en ce que** le poinçon (28) est bombé à son extrémité en contact avec la membrane.

9. Dispositif selon la revendication 1, **caractérisé en ce que** le dispositif (1) présente un support (6) et un couvercle (7) entre lesquels ou à partir desquels le canal (3) est formé.

10. Dispositif selon la revendication 9, **caractérisé en ce que** le dispositif comprend un élément de recouvrement (33) qui présente un poinçon (28).

11. Dispositif selon la revendication 10, **caractérisé en ce que** l'élément de recouvrement (33) est fixé au couvercle (7) lors de la fabrication du dispositif (1).

12. Dispositif selon l'une des revendications 10 ou 11 précédentes, **caractérisé en ce que** l'élément de recouvrement (33) présente latéralement au poinçon (28) une ouverture de passage (14) au travers de laquelle le fluide peut être intégré dans la zone d'acheminement (18) .

13. Dispositif selon l'une des revendications 11 ou 12 précédentes, **caractérisé en ce que** le poinçon (28) présente un alésage débouchant (32) au travers duquel le fluide échantillon (2) peut être introduit dans la zone d'acheminement (18).

14. Dispositif selon la revendication 13, **caractérisé en ce que** la paroi latérale (26) du poinçon présente sur la surface de paroi intérieure, des éléments conducteurs tels que des élévations (10).

15. Dispositif selon l'une des revendications 10 à 14 précédentes, **caractérisé en ce que** le poinçon (28) ou l'élément de recouvrement (33) sont conçus en tant qu'empreinte, dans laquelle la formation tridimensionnelle de la membrane (15) imprime sa forme.
